# EUROPEAN PATENT APPLICATION

(11) **EP 0 650 703 A1**
(43) Date of publication of application: **03.05.1995**
(21) Application number: 94401426.5
(22) Date of filing: 24.06.1994
(51) Int. Cl.: A61F 2/00

(54) **Adjustable periurethral sphincter**

(30) Priority: 06.07.1993 BR 9302774
(71) Applicant: Robert, Antoine Jean Henri, Rio de Janeiro (BR); Correia Lima, Salvador Vilar, Recife, Pernambuco (BR)
(72) Inventor: Robert, Antoine Jean Henri, Rio de Janeiro (BR); Correia Lima, Salvador Vilar, Recife, Pernambuco (BR)
(74) Representative: Burtin, Jean-François

(57) **Abstract**

The present invention refers to an adjustable periurethral tensor which consists of a one-piece element which does not require additional fittings that would bring about leakages. This enables the withdrawal and introduction of serum without additional surgical intervention and permits ideal control of the pressure on the urethra, thus aiding the sphincter to close.

The tensor consists of a supporting plate (3) having medical grade silicon layers and an inner reinforcement (4) made from polyester fibers such as Dacron^{(R)}. A hole (5), fastening pins (6) and an inflatable envelope (8) are provided in the supporting plate (3). The plate (3) is connected to a valve (9) through a connecting pipe (2).

## Description

The present invention refers to an adjustable periurethral tensor.

In the prior art, the solution envisaged for patients with sphincter functional problems was the use of an artificial sphincter comprised of an assembly made of several parts connected to one another. The most common example is a bag having predetermined sizes which is disposed around the sphincter. On filling same by means of a pump normally located in the scrotum or vagina, the bag shall be filled wih physiological serum, which will constrict the sphincter.

Said device poses several problems such as the bending of the connecting pipe between the constricting bag and the pump and the loss of initial serum due to the natural porosity of silicon, a material which is usually used and which cannot be replaced as it would require further surgery.

Additionally, in view of the great number of fittable parts, there may occur leakages and flaws in the numerous connections. Another serious problem detected is the tissue necrosis due to the overpressure exerted on the urethra.

In the prior art, a process is known for injecting Teflon^{(R).} (beads), collagen or other materials with the aim of artificially producing an obstruction. Said process has several drawbacks, namely, the difficulty in obtaining precision at the stage of injecting such materials; the impossibility of removing them subsequently even when it is not a surgical process, or regulating the intensity of the thus produced obstruction, as owing to the fact that an empirically determined amount of such materials is injected it is impossible to foresee with accuracy the final result of the obstruction.

The object of the present invention is to solve the problems of the art by providing a one-piece periurethral tensor which shall prevent any leakages.

Another object is to provide the tensor with a valve for allowing the withdrawal and injecting of physiological serum or any other suitable means, enabling a pressure control without the need of further surgery.

Another object is to provide a tensor which contrary to the ones in the current art that only allow for the sphincter to open and close, can help the sphincter to close, and enable to use it in patients lacking partial or total voluntary muscle control, for instance, paraplegics, patients with congenital or old age incontinence.

Anoher object is to provide a tensor which enables a precise control even in patients lacking total voluntary muscle control, wherein the patient himself may use the catheter without having to change the expanding device, thus providing the patient with more confidence and comfort and enabling him to enjoy a normal social life.

These and other objects shall be achieved in the present invention by providing an adjustable periurethral tensor consisting of a supporting plate having a hole in one end thereof and a plurality of fastening pins on its other end. The supporting plate is provided at the face opposite the one of the pins with an inflatable envelope which extends through a portion of the plate.

The present invention shall be better understood by reference to the attached drawings, wherein:

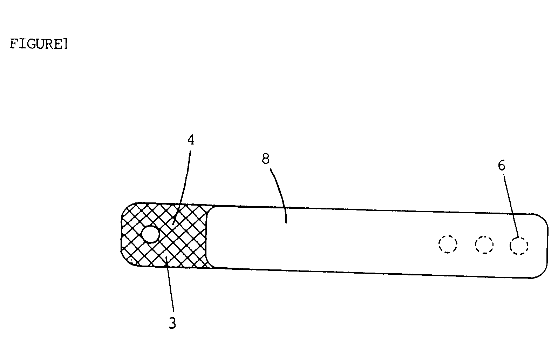

Figure 1 is a top view of the tensor, wherein the valve and the connecting pipe have been omitted, and

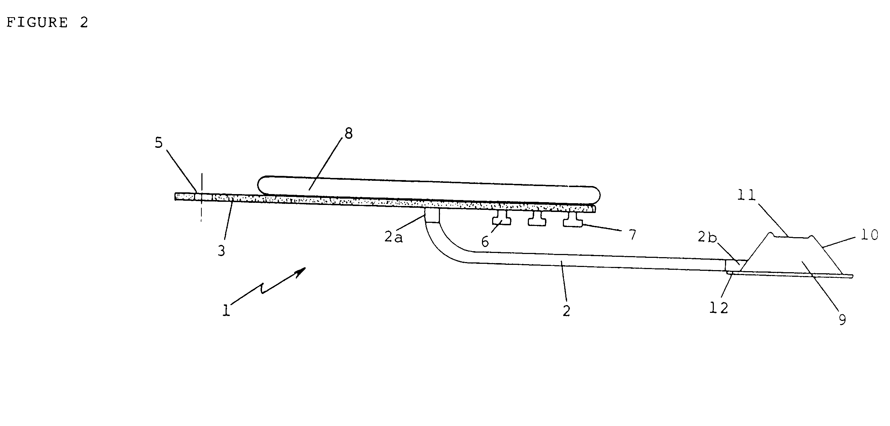

Figure 2 is a side view of the tensor, where it is possible to view the whole assembly.

With reference to the drawings, it is shown a tensor (1) basically comprised of two parts which are connected by a connecting pipe (2) usually of an inert flexible material such as silicon.

The first part consists of a supporting plate (3) comprised of layers of an inert flexible material, preferably medical grade silicon, having an inner reinforcement (4) of polyester fibres such as Dacron ^{(R).}

At one end of plate (3) a through-hole (5) is provided which is suitable for receiving one of the fastening pins (6) which may present a step (7) for providing a safer fastening of the tensor. Three holes or orifices are shown in the drawings for illustrative purposes only. The purpose of providing the tensor with a plurality of pins (6) is to have a one-size tensor which may be regulated according to the need, i.e according to the patient's biotype, thus enabling not only a more suitable adjusting to each individual but also a considerable reduction in the manufacturing and tensor application costs.

An inflatable envelope (8) is provided on the upper part of plate (3), for example, the above mentioned inert flexible material such as silicon whose flexibility is proper for expanding with the insertion of physiological serum or another suitable fluid from the connecting pipe (2) and a valve (9).

Since envelope (8) is disposed along the periphery of the urethra, it shall expand upon receiving the serum and this will effectively help to close the sphincter.

A fitting (2a) of the connecting pipe (2) which is also linked to valve (9) by a connection (2b) is provided in plate (3) adjacent pins (6). The fluid, preferably serum, shall be transferred from valve (9) to the inflatable envelope (8) through pipe (2), thus providing the precise and safe insertion and withdrawal of serum by means of valve (9).

The second part of the tensor is comprised of a valve (9) having an outer casing (10) with an inner reservoir for containing physiological serum which is inserted by a needle through the upper part (11) of valve (9). The upper part (11) and casing (10) are usually made of medical grade silicon. A core forming the abovementioned inner reservoir is provided in the inner part of valve (9). The core may be made from stainless steel, or preferably of ceramica or titanium which are harmless materials in a magnetic ressonance analysis.

Said valve is known in the prior art and US patent n° 4.673.394 may be cited as a reference thereto.

Said valve (9) is provided with a base (12) having orifices which allow the valve to be fastened thereto by means of suture. The valve is disposed under the abdomen skin so that the serum can be injected in a predetermined amount thus establishing an ideal and necessary pressure gradient according to the patient's conditions.

With such an arrangement, it is possible to withdraw and inject serum at any time without the need of surgery thus allowing for a constant pressure control.

The tensor of the present invention is suitable for people lacking partial or total voluntary muscle control and in cases of lack of total voluntary muscle control allows the patient himself to use a catheter without the need of any additional pressure adjustments.

It should be understood that minor changes may be possible provided that they do not depart from the scope of the claims appended hereto.

## Claims

1. An adjustable periurethral tensor (1) having a valve (9) for inserting and removing fluid and a connecting pipe (2) for conveying fluid, wherein the tensor is comprised of a supporting plate (3) having a hole (5) in an end thereof and a plurality of fastening pins (6) on its other end, the supporting plate (3) being provided with an inflatable envelope (8) on the opposite face of the pins (6), which envelope extends through a portion of the plate (3).

2. A tensor according to claim 1°, wherein the envelope (8) receives fluid through a valve (9) and pipe (2), the pipe (2) being connected to the plate (3) by a fitting (2a) and to the valve (9) by a connection (2b).

3. A tensor according to claim 1°, wherein the valve (9) is provided with an outer casing (10) and a core which define a reservoir suitable for receiving the fluid which is to be transferred to the envelope (8).

4. A tensor according to claim 1°, wherein each of the fastening pins (6) is provided with a step (7) for fitting in the hole (5), the fitting of the pins (6) in the hole (5) being variable due to the tensor adjustment.

5. A tensor according to claim 1°, wherein the plate (3) is provided with layers of an inert flexible material having an inner reinforcement (4) made of polyester fibres such as Dacron^{(R).}.

6. A tensor according to claim 1, 2, 3, 4 or 5° wherein the tensor (1) is fully made from an inert flexible material, preferably a medical grade silicon.
